(19) Europäisches Patentamt
European Patent Office
Office européen des brevets

(11) Publication number: **0 267 976 B1**

(12) # EUROPEAN PATENT SPECIFICATION

(45) Date of publication of the patent specification: **29.08.90**

(51) Int. Cl.5: **C07D 498/04,** C07D 519/00 // (C07D498/04, 263:00, 263:00),(C07D519/00, 498:00, 498:00)

(21) Application number: **86115932.5**

(22) Date of filing: **17.11.86**

(54) **An improved process for the preparation of bicyclic amide acetals.**

(43) Date of publication of application: **25.05.88 Bulletin 88/21**

(45) Publication of the grant of the patent: **29.08.90 Bulletin 90/35**

(84) Designated Contracting States: **AT BE CH DE FR GB IT LI LU NL SE**

(56) References cited:
**DE-A- 2 344 607**
**FR-A- 1 518 386**

(73) Proprietor: **Ashland Oil, Inc., 1000 Ashland Drive, Russell Kentucky 41169(US)**

(72) Inventor: **Goel, Anil B., 373 Eastworth Court, Worthington Ohio 43085(US)**
Inventor: **Richards, Harvey J., 2210 Bristol Road, Columbus Ohio 43221(US)**

(74) Representative: **Vossius & Partner, Siebertstrasse 4 P.O. Box 86 07 67, D-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 267 976 B1

## Description

This invention relates to an improved process for the preparation of bicyclic amide acetals by the reaction of an organic nitrile with a dialkanol amine in the presence of an alkaline catalyst wherein the reaction temperature is maintained below about 140°C and the bicyclic amide acetal is removed from the reaction mixture by extraction in a hydrocarbon solvent.

The synthesis of bicyclic amide acetals by the reaction of a dialkanol amine, such as diethanol amine with alkyl cyanides has been reported to result in relatively low yields (30-40%) in Angew. Chem. 85, (1973). A similar process is disclosed in German Patent Publication No. 2 344 607. Reaction temperatures used in this synthesis have been in the range of 120 to 180°C.

The preparation and reactions of bicyclic amide acetals are also described in Synthesis, (1971), pp. 16–26.

Although low yield synthesis of bicyclic amide acetals at 120 to 180°C usually in the presence of a catalyst such as an alkali metal, has been described, no reaction between the dialkanol amine and the bicyclic amide acetal at these temperatures has been disclosed. Because all of the bicyclic amide acetals boil at temperatures greater than 170°C under ambient pressure, and at these temperatures the dialkanol amine reacts with the bicyclic amide acetal, lower yields in the prior art process were inevitable. The use of solvent extraction to separate the bicyclic amide acetal from the reaction mixture to produce significantly higher yields has not previously been disclosed or suggested. The extraction solvent can be any inert solvent and can even include an excess of the starting nitrile itself.

The bicyclic amide acetals produced by the improved process of this invention include those having the Formula I

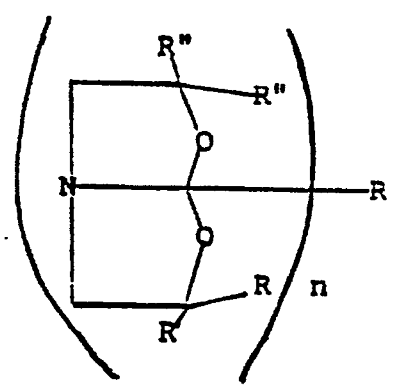

I

wherein n is 1 or 2, R and R″ independently represent hydrogen, an alkyl group containing from 1 to 10 carbon atoms or an aryl group containing from 6 to 12 carbon atoms, and when n is 1, R′ represents an alkyl group containing from 1 to 20 carbon atoms, an aryl group containing form 6 to 14 carbon atoms, or an alkaryl group containing from 7 to 20 carbon atoms, and when n is 2, R′ represents an alkylene group containing from 1 to 20 carbon atoms or an arylene group containing form 6 to 14 carbon atoms or an alkarylene group containing from 7 to 20 carbon atoms.

It has been found that higher yields of purified bicyclic amide acetals can be obtained by separating the bicyclic amide acetal via solvent extraction prior to any distillation of the bicycle amide acetal.

Thus, following the reaction of diethanol amine with an alkyl cyanide at a reaction between room temperature and 140°C, the reaction mixture in our process is subjected to extraction at about room temperature to isolate the bicyclic amide acetal product. The preferred solvents for the extraction of bicyclic amide acetal from the reaction mixture are hydrocarbons and hydrocarbon ethers containing from 4 to 20 carbon atoms and most preferred are aliphatic hydrocarbons such as pentane, hexane, heptane, petroleum ether and aromatic hydrocarbons such as benzene and toluene. The starting nitriles themselves can also be used as extraction solvents in our invention. In the extraction the bicyclic amide acetals selectively go into the solvent layer and dialkanol amine and catalyst as well as high boiling byproducts remain in the remainder of the reaction mixture. The extracted bicyclic amide acetal, along with the nitrile and solvent can then be subjected to fractional distillation at any desired temperature without loss of yield. The use of an excess amount of the nitrile often obviates the necessity for any other solvent. The nitrile containing the bicyclic amide acetal separates and can be isolated readily from the remainder of the reaction mixture.

The organic nitriles useful in this process include alkane mononitriles having from 1 to 20 carbon atoms in the aliphatic chain, aromatic mononitriles having from 7 to 15 carbon atoms and alkylaromatic mononitriles having from 8 to 21 carbon atoms and alkane dinitriles having from 3 to 22 carbon atoms, aromatic dinitriles having from 8 to 16 carbon atoms and alkylaromatic dinitriles having from 9 to 22 carbon atoms.

The dialkanol amines useful in the process of this invention include substituted and unsubstituted dialkanol amines having the general formula $HOC(R)_2CH_2NHCH_2C(R'')_2OH$ wherein R and R″ have the foregoing designations.

While the above mentioned side reaction is demonstrated in reference examples 1 and 2 and the preleminary experiments are documented in reference examples 3 and 4, the process of this invention is further illustrated in the following representative examples 1–5.

REFERENCE EXAMPLE 1

Diethanol amine (0.26 g) and a bicyclic amide acetal of Formula I wherein n is 1, R and R″ are hydrogen and R′ is benzyl (0.51 g) were mixed and heated in a closed mini reactor with constant stirring at 160°C for 2½ hours. GLC analysis of the mixture showed that 47% of the diethanol amine and about

55% of the bicyclic amide acetal had reacted (were no longer present).

REFERENCE EXAMPLE 2

Diethanol amine (1.08 g) and a bicyclic amide acetal of Formula I where n is 1, R and R″ are hydrogen and R′ is methyl (1.3 g) were mixed in a mini reactor and heated at 120°C for three hours, followed by heating at 150°C for two hours. GLC analysis of the mixture indicated that about 20% by weight of the starting diethanol amine and 25% of the bicyclic amide acetal had reacted.

REFERENCE EXAMPLE 3

Diethanol amine (1.05 g) and the bicyclic amide acetal of Formula I in which n is 1, R and R″ are hydrogen and R′ is methyl (1.25 g) were mixed and heated at 120°C for three hours. GLC analysis showed the consumption of only about 2% by weight of each reactant.

REFERENCE EXAMPLE 4

The procedure of the prior art (German Patent Publication No. 2 344 607) was followed using 117.15 g of benzyl cyanide and 105.14 g of diethanol amine. The reaction was carried out at 140°C for about 20 hours. The GLC analysis of the reaction mixture indicated the presence of about 65% by weight of the product of Formula I wherein n is 1, R and R″ are hydrogen and R′ is benzyl and about 10% by weight of benzyl cyanide and 4% of diethanol amine. The reaction mixture was subjected to fractional distillation under reduced pressure. Because of the high boiling nature of the product, the pot temperature had to be increased about 150°C in order to distill the product at 110–20°C/0.6–2 mm Hg pressure which afforded a 41% overall yield of the bicyclic amide acetal. The pot residue (95 g, 43%) was found to contain only traces of the product bicyclic amide acetal and was highly viscous indicating higher molecular weight byproducts. This shows that about 14% by weight of the bicyclic amide acetal product was lost during the isolation by distillation.

EXAMPLE 1

Diethanol amine (25.6 g) containing 2 mole percent of sodium and benzonitrile (25 g) were mixed and heated at 100°C for 56 hours under nitrogen. The GLC analysis of the reaction mixture indicated the formation of about 38% by weight of bicyclic amide acetal of Formula I wherein n is 1, R and R″ are hydrogen and R′ is phenyl. The reaction mixture was brought back to room temperature and the product and the unreacted benzonitrile were separated from diethanol amine and the catalyst by extraction with three 40 ml portions of hexane and 20 ml of toluene. GLC analysis of the combined extracts showed them to contain mainly the nitrile and the bicyclic amide acetal product with only traces (less than 1%) of diethanol amine, whereas the residue was found to be mainly diethanol amine with traces (1 to 2%) of bicyclic amide acetal and nitrile. The solvent was removed from the extract and the residue was fractionally distilled giving a 35% yield of the bicyclic amide acetal (78–79°C/0.02 mm Hg). This experiment demonstrates that essentially no loss of product occurs when separation of product by extraction is carried out prior to the distillation step which precludes possible reaction between the bicyclic amide acetal and diethanol amine.

EXAMPLE 2

The procedure of Example 1 was repeated using 52.7 g of amine with 2 mole percent of sodium and 90.7 g of undecyl cyanide. GLC analysis of the reaction mixture after 72 hours of reaction at 100°C showed the formation of about 42% of the bicyclic amide acetal of Formula I in which n is 1, R and R″ are hydrogen and R′ is undecyl. The extraction with pentane (three 50 ml portions) and fractional distillation of the extract afforded about 52 g (greater than 38% yield) of the bicyclic amide acetal product.

EXAMPLE 3

A 40 g mixture containing approximately 50% by weight of diethanol amine, 45% of a bicyclic amide acetal of Formula I wherein n is 1, R and R″ are hydrogen and R′ is methyl and 5% of acetonitrile was extracted with three 80 ml portions of pentane at room temperature. The extracts were combined and analyzed and it was found that 95% of the total bicyclic amide acetal had been extracted. The GLC analysis of the residue not extracted showed the presence mainly of diethanol amine with about 5% of the bicyclic amide acetal.

EXAMPLE 4

A mixture of 20% by weight of the bicyclic amide acetal of Example 3 and 80% of diethanol amine was prepared. The bicyclic amide acetal was separated by extraction using acetonitrile as the solvent as in Example 3. GLC analysis of the residue left after extraction indicated the presence of 95% of diethanol amine and about 5% of the bicyclic amide acetal.

EXAMPLE 5

Acetonitrile (83.8 g, 2.04 mols) and diethanolamine (88.2 g, 0.84 mol) containing 2 mole% of sodium (based on diethanol amine) as catalyst were reacted for 30 hours at 79°C to produce the methyl bicyclic amide acetal of Formula I wherein n is 1, R and R″ are hydrogen and R′ is methyl in 75% yield. Separation of the bicyclic amide acetal product from the unreacted starting material and catalyst was accomplished with pentane solvent. The pentane extracted 93.5% of the bicyclic amide acetal and no diethanol amine (GLC analysis). The mother liquor left after the extraction was found by GLC to contain 100% of the unreacted diethanol amine and only 6.5% of the bicyclic amide acetal.

## Claims

1. A process for preparing a bicyclic amide acetal from an organic nitrile and a dialkanol amine characterized by conducting the reaction at a temperature in the range of from room temperature to 140°C and then extracting the bicyclic amide acetal product from the reaction mixture with a solvent.

2. The process of Claim 1 wherein the bicyclic amide acetal is one conforming to the formula

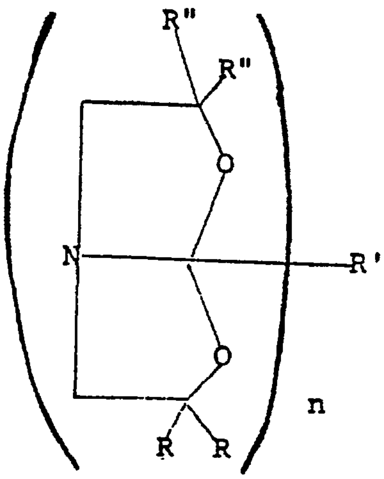

wherein n is 1 or 2, and R and R″ independently represent hydrogen, an alkyl group containing from 1 to 10 carbon atoms or and aryl group containing from 6 to 12 carbon atoms, and when n is 1, R′ represents and alkyl group containing from 1 to 20 carbon atoms, an aryl group containing from 6 to 14 carbon atoms, or an alkaryl group containing from 7 to 20 carbon atoms and when n is 2, R′ represents an alkylene group containing from 1 to 20 carbon atoms, an arylene group containing from 6 to 14 carbon atoms or an alkarylene group containing from 7 to 20 carbon atoms.

3. The process of Claim 2 wherein the organic nitrile is one selected from the group consisting of an alkane mononitrile having from 1 to 20 carbon atoms in the aliphatic chain, an allkylaromatic mononitrile having from 8 to 21 carbon atoms, an alkane dinitrile having from 3 to 22 carbon atoms, an aromatic dinitrile having from 8 to 16 carbon atoms and alkylaromatic dinitrile having from 9 to 22 carbon atoms.

4. The process of Claim 3 wherein the dialkanol amine is one which has the formula

$HOC(R)_2CH_2NHCH_2C(R'')_2OH$.

5. The process of Claim 4 wherein the solvent is a member selected from the group consisting of the organic nitrile and a hydrocarbon containing from 2 to 20 carbon atoms.

6. The process of Claim 4 wherein the organic nitrile is benzyl cyanide, the dialkanol amine is diethanol amine and the solvent is hexane and toluene.

7. The process of Claim 4 wherein the organic nitrile is undecyl cyanide, the dialkanol amine is diethanol amine and the solvent is pentane.

8. The process of Claim 4 wherein the organic nitrile is acetonitrile, the dialkanol amine is diethanol amine and the solvent pentane and acetonitrile.

9. The process of Claim 4 wherein the organic nitrile is acetonitrile, the dialkanol amine is diethanol amine and the solvent is acetonitrile.

10. The process of Claim 4 wherein the organic nitrile is acetonitrile, the dialkanol amine is diethanol amine and the solvent is pentane.

## Patentansprüche

1. Verfahren zur Herstellung eines bicyclischen Amidacetals aus einem organischen Nitril und einem Dialkanolamin in Gegenwart eines alkalischen Katalysators, dadurch gekennzeichnet, daß die Reaktion bei einer Temperatur von Raumtemperatur bis 140°C ausgeführt wird und dann das bicyclische Amidacetal-Produkt mit einem Lösungsmittel aus dem Reaktionsgemisch extrahiert wird.

2. Verfahren nach Anspruch 1, in dem das bicyclische Amidacetal nachfolgende allgemeine Formel besitzt

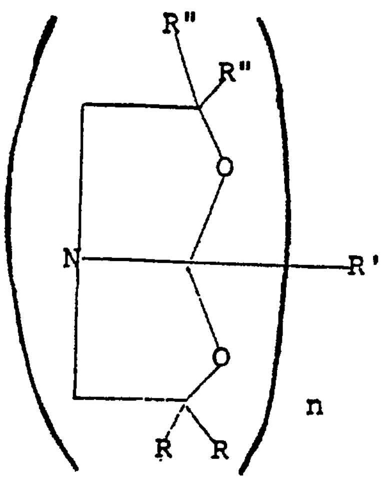

in der n 1 oder 2 ist, R und R″ jeweils unabhängig ein Wasserstoffatom, einen $C_{1-10}$-Alkylrest oder einen $C_{6-12}$-Arylrest darstellen, und wenn n 1 ist, R′ einen $C_{1-20}$-Alkylrest, einen $C_{6-14}$-Arylrest oder einen $C_{7-20}$-Alkarylrest darstellt und wenn n 2 ist, R′ einen $C_{1-20}$-Alkylenrest, einen $C_{6-14}$-Arylenrest oder einen $C_{7-20}$-Alkarylenrest darstellt.

3. Verfahren nach Anspruch 2, in dem das organische Nitril ein Alkan-mononitril mit 1 bis 20 Kohlenstoffatomen in der aliphatischen Kette, ein aromatisches Mononitril mit 7 bis 15 Kohlenstoffatomen, ein alkylaromatisches Mononitril mit 8 bis 21 Kohlen stoffatomen, ein Alkandinitril mit 3 bis 22 Kohlenstoffatomen, ein aromatisches Dinitril mit 8 bis 16 Kohlenstoffatomen und ein alkylaromatisches Dinitril mit 9 bis 22 Kohlenstoffatomen sein kann.

4. Verfahren nach Anspruch 3, in dem das Dialkanolamin nachstehende Formel

$HOC(R)_2CH_2NHCH_2C(R'')_2OH$

besitzt.

5. Verfahren nach Anspruch 4, in dem das Lösungsmittel ein organisches Nitril oder ein Kohlenwasserstoff mit 2 bis 20 Kohlenstoffatomen ist.

6. Verfahren nach Anspruch 4, in dem das organische Nitril Benzylcyanid ist, das Dialkanolamin Diethanolamin, und das Lösungsmittel Hexan und Toluol ist.

7. Verfahren nach Anspruch 4, in dem das organische Nitril Undecylcyanid, das Dialkanolamin Diethanolamin und das Lösungsmittel Pentan ist.

8. Verfahren nach Anspruch 4, in dem das organische Nitril Acetonitril, das Dialkanolamin Diethanolamin und das Lösungsmittel Pentan und Acetonitril ist.

9. Verfahren nach Anspruch 4, in dem das organische Nitril Acetonitril, das Dialkanolamin Diethanolamin und das Lösungsmittel Acetonitril ist.

10. Verfahren nach Anspruch 4, in dem das organische Nitril Acetonitril, das Dialkanolamin Diethanolamin und das Lösungsmittel Pentan ist.

## Revendications

1. Procédé pour la fabrication d'un acétal d'amide bicyclique à partir d'un nitrile organique et d'une dialcanolamine en présence d'un catalyseur alcalin, caractérisé en ce qu'on effectue la réaction à une température dans l'intervalle de la température ambiante à 140°C et on extrait ensuite l'acétal d'amide bicyclique du mélange de réaction par un solvant.

2. Le procédé selon la revendication 1, dans lequel l'acétal d'amide bicyclique répond à la formule:

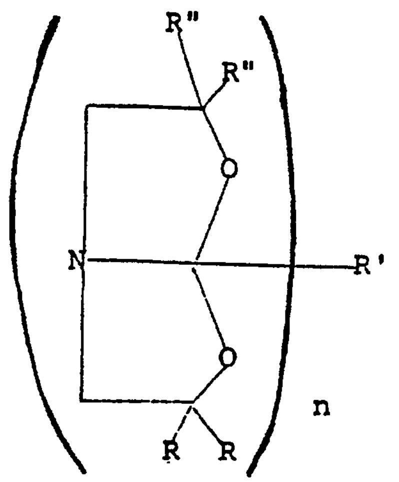

dans laquelle n est égal à 1 ou 2, R et R″ représentent chacun indépendamment l'hydrogène ou un groupe alkyle en $C_1$–$C_{10}$ ou un groupe aryle en $C_6$–$C_{12}$ et, lorsque n'est égal à 1, R′ représente un groupe alkyle en $C_1$–$C_{20}$, un groupe aryle en $C_6$–$C_{14}$ ou un groupe alkylaryle en $C_7$–$C_{20}$ et, lorsque n est égal à 2, R′ représente un groupe alkylène en $C_1$–$C_{20}$, un groupe arylène en $C_6$–$C_{14}$ ou un groupe alkylarylène en $C_7$–$C_{20}$.

3. Le procédé selon la revendication 2, dans lequel le nitrile organique est choisi parmi un alcanemononitrile à chaîne aliphatique en $C_1$–$C_{20}$, un mononitrile aromatique en $C_7$–$C_{15}$, un mononitrile alkylaromatique en $C_8$–$C_{21}$, un alcanedinitrile en $C_3$–$C_{22}$, un dinitrile aromatique en $C_8$–$C_{16}$ et un dinitrile alkyl aromatique en $C_9$–$C_{22}$.

4. Le procédé selon la revendication 3, dans lequel la dialcanolamine répond à la formule $HOC(R)_2CH_2NHCH_2C(R'')_2OH$.

5. Le procédé selon la revendication 4, dans lequel le solvant est choisi parmi le nitrile organique et un hydrocarbure en $C_2$–$C_{20}$.

6. Le procédé selon la revendication 4, dans lequel le nitril organique est le cyanure de benzyle, la dialcanolamine et la diéthanolamine et le solvant est l'hexane et le toluène.

7. Le procédé selon la revendication 4, dans lequel le nitrile organique est le cyanure d'undécyle, la dialcanolamine est la diéthanolamine et le solvant est le pentane.

8. Le procédé selon la revendication 4, dans lequel le nitrile organique est l'acétonitrile, la dialcanolamine est la diéthanolamine et le solvant est le pentane et l'acétonitrile.

9. Le procédé selon la revendication 4, dans lequel le nitrile organique est l'acétonitrile, la dialcanolamine est la diéthanolamine et le solvant es l'acétonitrile.

10. Le procédé selon la revendication 4, dans lequel le nitrile organique est l'acétonitrile, la dialcanolamine est la diéthanolamine et le solvant est le pentane.